# EUROPEAN PATENT APPLICATION

(11) **EP 0 680 960 A1**
(43) Date of publication of application: **08.11.1995**
(21) Application number: 94903008.4
(22) Date of filing: 15.12.1993
(51) Int. Cl.: C07D 401/04, C07D 403/04

(54) **IMIDAZOLE COMPOUND**

(30) Priority: 25.01.1993 JP 27145/93; 25.01.1993 JP 27146/93
(71) Applicant: THE GREEN CROSS CORPORATION, Osaka-shi Osaka 541 (JP)
(72) Inventor: YANAI, Kazuhiko, Sendai-shi Miyagi 989-32 (JP); WATANABE, Takehiko, Sendai-shi Miyagi 981 (JP); GOTOH, Tomokazu The Green Cross Corporation, Hirakata-shi, Osaka 573 (JP); SAKASHITA, Hiroshi The Green Cross Corporation, Hirakata-shi, Osaka 573 (JP); MURAKAMI, Kazuki The Green Cross Corporation, Hirakata-shi, Osaka 573 (JP); SUGIURA, Masanori The Green Cross Corporation, Hirakata-shi, Osaka 573 (JP); FUKAYA, Chikara The Green Cross Corporation, Hirakata-shi, Osaka 573 (JP)
(74) Representative: Hansen, Bernd, Dr. Dipl.-Chem.
(86) International application number: JP9301822
(87) International publication number: WO9417058

(57) **Abstract**

The invention aims at providing a novel compound having vitamin H₃ receptor antagonism and relates to a compound represented by general formula (O) or pharmaceutically acceptable salts thereof, wherein m represents an integer of 4 to 6; R₁ represents hydrogen, lower alkyl or aralkyl; R₂ and R₃ may be the same or different from each other and each represents hydrogen or lower alkyl; R₄ represents hydrogen, linear or branched alkyl, cycloalkyl, cycloalkylalkyl, optionally substituted aryl, or optionally substituted aralkyl; Z represents R₅ or A-R₆; A represents S or O; R₅ represents hydrogen, lower alkyl, optionally substituted aryl, or optionally substituted aralkyl; and R₆ represents lower alkyl, lower alkenyl, lower alkynyl, or optionally substituted aralkyl.

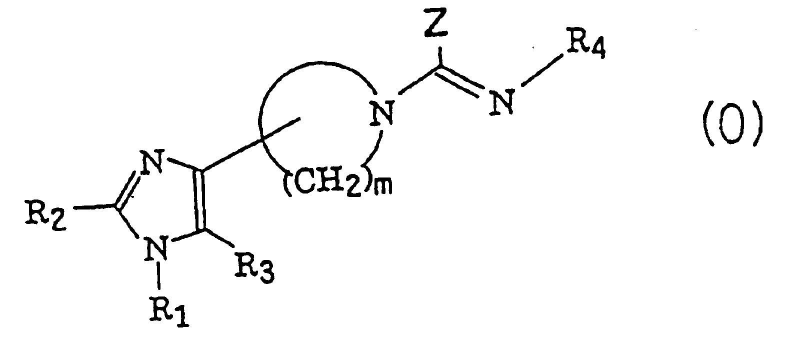

## Description

### Technical Field

This invention relates to an imidazole-series compound which is useful as a histamine H₃ receptor antagonist.

### Background Art

Histamine, which is a physiologically active endogenous compound occurring in mammals, exerts its effects through an interaction with a specific site called a receptor. Such receptors of one type have been known as histamine H₁ receptors [Ash and Schild, Brit. J.Pharmac. Chemother., 27, 427 (1966)]. The effects of histamine mediated by such a receptor are blocked by an H₁ antagonist, for example, mepyramine. Receptors of the second type are known as histamine H₂ receptors (Black et al., Nature, (1972), 236, 385]. The effects of histamine mediated by these receptors are not blocked by mepyramine but by H₂ antagonists such as burimamide or cimetidine.

In recent years, studies on the central histamine neuron system have proceeded, and it has been thus clarified that the histamine neuron system affects the central nervous system over a wide range. It has been revealed so far that histamine regulates brain functions, in particular, hypothalamic functions in the central region (sleep/vigilance rhythm, internal secretion, eating/drinking behavior, sexual behavior, etc.). It has also been clarified that a histamine H₃ receptor, which is an autorecetpor, exists in the presynaptic membrane of the neuron.

The histamine H₃ receptor of the third type has been identified [for example, Arrang et al., Nature (1987), 327, 117; and Van der Werf et al., Trends Pharmacol. Sci., 10, 159 (1989)]. This receptor is stimulated by an H₃ agonist such as (R)-α-methylhistamine and blocked by an H₃ antagonist such as thioperamide.

It is known that the histamine H₃ receptor controls the histamine level in the brain, and a histamine H₃ receptor antagonist can elevate the histamine level in the brain. Moreover, it has been reported that histamine regulates the release of acetylcholine, noradrenalin, serotonin, etc. from the nerve ending via the histamine H₃ receptor.

In addition to the thioperamide as described above, compounds having histamine H₃ receptor antagonism are described in, for example, JP-A-61-267574 and EP-A-494010.

Under these circumstances, the present inventors have further conducted studies and, as a result, succeeded in the synthesis of novel compounds having histamine H₃ receptor antagonism, thus completing the present invention.

### Disclosure of the Invention

The present invention relates to a compound represented by the following general formula (0) [hereinafter referred to as the invention compound (0)]: 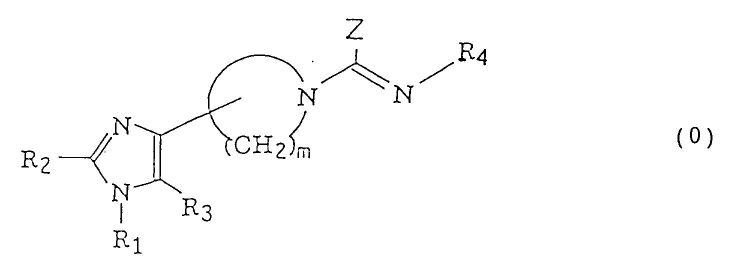
wherein m represents an integer of from 4 to 6; R₁ represents a hydrogen atom or a lower alkyl or aralkyl group; R₂ and R₃ may be either the same or different from each other and each represents a hydrogen atom or a lower alkyl group; R₄ represents a hydrogen atom, a linear or branched alkyl group, a cycloalkyl group, a cycloalkylalkyl group, a substituted or unsubstituted aryl group or a substituted or unsubstituted aralkyl group; and Z represents R₅ or A-R₆, wherein A represents S or O, R₅ represents a hydrogen atom, a lower alkyl group, a substituted or unsubstituted aryl group or a substituted or unsubstituted aralkyl group, and R₆ represents a lower alkyl group, a lower alkenyl group, a lower alkynyl group or a substituted or unsubstituted aralkyl group; or a pharmaceutically acceptable salt thereof.

Accordingly, the present invention relates to a compound represented by the following general formula (1) [hereinafter referred to as the invention compound (1)]: 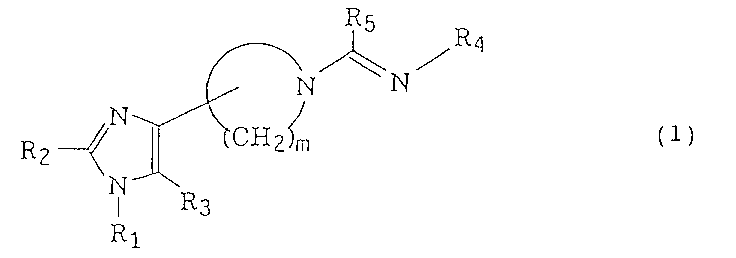
wherein m and R₁ to R₅ are each as defined above; and a pharmaceutically acceptable salt thereof.

The present invention further relates to a compound represented by the following general formula (2) [hereinafter referred to as the invention compound (2)]: 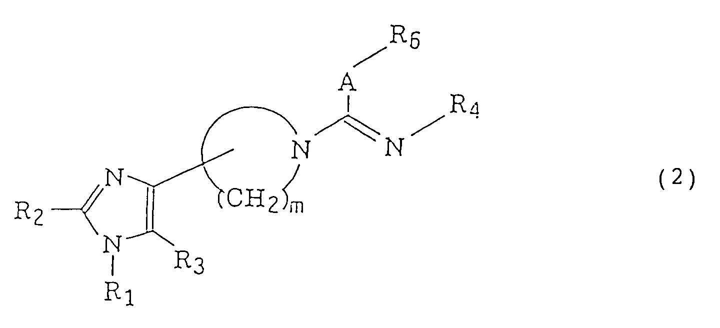
wherein A, m, R₁ to R₄ and R₆ are each as defined above; and a pharmaceutically acceptable salt thereof.

When the compound of the general formula (0) occurs in the form of tautomers, these tautomers are also included in the scope of the present invention.

When the compound of the general formula (0) has optical isomers, these optical isomers are also included in the scope of the present invention.

Now, the terms employed in the description of the substituents R₁ to R₆, involving those used in common to R₁ to R₆, will be illustrated.

The lower alkyl groups are preferably linear or branched alkyl groups having 1 to 6 carbon atoms. Specific examples thereof include methyl, ethyl, n-propyl, iso-propyl, n-butyl, iso-butyl, sec-butyl, tert-butyl, n-pentyl and n-hexyl groups.

The linear or branched alkyl groups are preferably those having 1 to 8 carbon atoms. Specific examples thereof include methyl, ethyl, n-propyl, iso-propyl, n-butyl, iso-butyl, sec-butyl, tert-butyl, n-pentyl, n-hexyl, 1,1-dimethylpropyl, 1,2-dimethylpropyl and 1,2,2-trimethylpropyl groups.

The cycloalkyl groups are preferably those having 3 to 10 carbon atoms. The cycloalkyl groups include not only monocycloalkyl groups (for example, cyclopentyl, cyclohexyl and cycloheptyl) but also polycycloalkyl groups (for example, bicycloalkyl and tricycloalkyl). Examples of the bicycloalkyl groups include norbornyl (for example, exo-2-norbornyl and endo-2-norbornyl), 3-pinanyl and bicyclo[2.2.2]oct-2-yl groups, while examples of the tricycloalkyl groups include adamantyl groups (for example, 1-adamantyl and 2-adamantyl). Such a cycloalkyl group may be substituted by alkyl group(s), etc.

The cycloalkylalkyl groups are preferably those composed of a cycloalkyl group having 3 to 10 carbon atoms with a linear or branched alkyl group having 1 to 3 carbon atoms. Specific examples thereof include 1-cyclohexylethyl and 1-cyclopropylethyl groups.

The lower alkenyl groups are preferably linear or branched alkenyl groups having 3 to 6 carbon atoms. Specific examples thereof include allyl, 1-methyl-2-propenyl, 2-methyl-2-propenyl, cis-2-butenyl, trans-2-butenyl and 3-methyl-2-butenyl groups.

The lower alkynyl groups are preferably those having 3 to 6 carbon atoms. A specific example thereof includes a 2-propynyl group.

The substituted aryl groups are preferably phenyl and naphthyl groups which may be substituted by halogen atoms and trifluoromethyl, lower alkyl, lower alkoxy, lower alkylthio, cyano and nitro groups.

Specific examples thereof include phenyl, 1-naphthyl, 2-chlorophenyl, 3-chlorophenyl, 4-chlorophenyl, 4-trifluoromethylphenyl, 3-fluorophenyl, 4-fluorophenyl, 2-methoxyphenyl, 4-methoxyphenyl, 2-tolyl and 3-tolyl groups.

The aralkyl groups are preferably benzyl and trityl groups.

The substituted aralkyl groups are preferably arylalkyl groups composed of a phenyl or naphthyl group, which may be substituted by halogen atoms and trifluoromethyl, lower alkyl, lower alkoxy, lower alkylthio, cyano and nitro groups, and a linear or branched alkyl group having 1 to 4 carbon atoms.

Specific examples thereof include benzyl, α-methylbenzyl, phenethyl, 3-phenylpropyl, 4-phenylbutyl, 4-chlorobenzyl, 4-fluorobenzyl, 4-methoxybenzyl, 4-chloro-α-methylbenzyl, 4-fluoro-α-methylbenzyl and 4-methoxy-α-methylbenzyl groups.

Among the compounds represented by the general formula (0), preferable examples include those wherein:
m is from 4 to 6;
R₁ is a hydrogen atom, an alkyl group having 1 to 6 carbon atoms or an aralkyl group carrying an alkyl moiety having 1 to 4 carbon atoms;
R₂ and R₃ are each a hydrogen atom or an alkyl group having 1 to 6 carbon atoms;
R₄ is a hydrogen atom; a linear or branched alkyl group having 1 to 8 carbon atoms, a cycloalkyl group having 3 to 10 carbon atoms, a cycloalkylalkyl group composed of a cycloalkyl moiety having 3 to 10 carbon atoms and an alkyl moiety having 1 to 3 carbon atoms, a substituted or unsubstituted aryl group or a substituted or unsubstituted aralkyl group carrying an alkyl moiety having 1 to 4 carbon atoms;
R₅ is a hydrogen atom, an alkyl group having 1 to 6 carbon atoms, a substituted or unsubstituted aryl group or a substituted or unsubstituted aralkyl group carrying an alkyl moiety having 1 to 4 carbon atoms; and
R₆ is an alkyl group having 1 to 6 carbon atoms, an alkenyl group having 3 to 6 carbon atoms, an alkynyl group having 3 to 6 carbon atoms or a substituted or unsubstituted aryl group.

Preferable examples of the compounds represented by the general formula (0) are those satisfying the following requirements:
(1) A compound wherein m is 5 and R₁, R₂ and R₃ are each a hydrogen atom.
(2) A compound wherein R₄ is a cycloalkyl group, such as monocycloalkyl, bicycloalkyl and tricycloalkyl groups. A preferable example of the monocycloalkyl group is a cyclohexyl group. A preferable example of the bicycloalkyl group is a norbornyl group, more preferably a 2-exo-norbornyl group. A preferable example of the tricycloalkyl group is an adamantyl group, more preferably a 1-adamantyl group.
(3) A compound wherein R₄ is a substituted or unsubstituted phenyl group or a substituted or unsubstituted phenylalkyl group.
(4) A compound wherein R₅ is a hydrogen atom.
(5) A compound wherein A is S and R₆ is a lower alkyl group.
(6) A compound wherein a lower alkyl group is a methyl group.

Preferable examples of the compound represented by the general formula (1) are as follows:
N-cyclohexyl-N',N'-[1,5-[3-(4(5)-1H-imidazolyl)pentanediyl]]formamidin;
N-(1-adamantyl)-N',N'-[1,5-[3-(4-(5)-1H-imidazolyl)pentanediyl]]formamidin;
N-(exo-2-norbornyl)-N',N'-[1,5-[3-(4(5)-1H-imidazolyl)pentanediyl]]formamidin;
N-(2,2-dimethyl-1-methylpropyl)-N',N'-[1,5-[3-(4(5)-1H-imidazolyl)pentanediyl]]formamidin;
N-(4-chlorobenzyl)-N',N'-[1,5-[3-(4(5)-1H-imidazolyl)pentanediyl]]formamidin;
N-(phenethyl)-N',N'-[1,5-[3-(4(5)-1H-imidazolyl)pentanediyl]]formamidin;
N-(3-fluorophenyl)-N',N'-[1,5-[3-(4(5)-1H-imidazolyl)pentanediyl]]formamidin; and
N-phenyl-N',N'-[1,5-[3-(4(5)-1H-imidazolyl)pentanediyl]]formamidin.

Preferable examples of the compound represented by the general formula (2) are as follows:
N-cyclohexyl-N',N'-[1,5-[3-(4(5)-1H-imidazolyl)pentanediyl]]-S-methylisothiourea;
N-(1-adamantyl)-N',N'-[1,5-[3-(4(5)-1H-imidazolyl)pentanediyl]]-S-methylisothiourea;
N-(exo-2-norbornyl)-N',N'-[1,5-[3-(4(5)-1H-imidazolyl)pentanediyl]]-S-methylisothiourea;
N-(2,2-dimethyl-1-methylpropyl)-N',N'-[1,5-[3-(4(5)-1H-imidazolyl)pentanediyl]]-S-methylisothiourea;
N-(4-chlorobenzyl)-N',N'-[1,5-[3-(4(5)-1H-imidazolyl)pentanediyl]]-S-methylisothiourea;
N-(phenethyl)-N',N'-[1,5-[3-(4(5)-1H-imidazolyl)pentanediyl]]-S-methylisothiourea;
N-(3-fluorophenyl)-N',N'-[1,5-[3-(4(5)-1H-imidazolyl)pentanediyl]]-S-methylisothiourea; and
N-phenyl-N',N'-[1,5-[3(4(5)-1H-imidazolyl)pentanediyl]]-S-methylisothiourea.

These compounds may form pharmaceutically acceptable acid addition salts together with acids such as hydrochloric acid, hydrobromic acid, hydroiodic acid, sulfuric acid, phosphoric acid, acetic acid, citric acid, maleic acid, lactic acid, ascorbic acid, fumaric acid, oxalic acid, methanesulfonic acid, ethanesulfonic acid and p-toluenesulfonic acid.

Now methods for synthesizing the invention compound (1) will be described in detail.
(1) 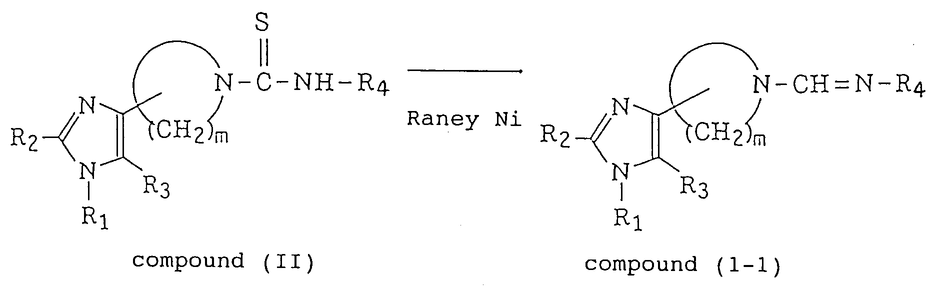 wherein each substituent is as defined above.
   The compound (II) is a publicly known compound described in, for example, JP-A-61-267574 and EP-A-494010.
   The compound (II) is reduced by using a catalyst, for example, Raney nickel. Thus, the invention compound (1-1) can be obtained. This reaction is effected in, for example, ethanol. The reaction conditions are exemplified by a reaction temperature ranging from ice-cooling to room temperature and a reaction time ranging from 10 minutes to 5 hours.
(2) 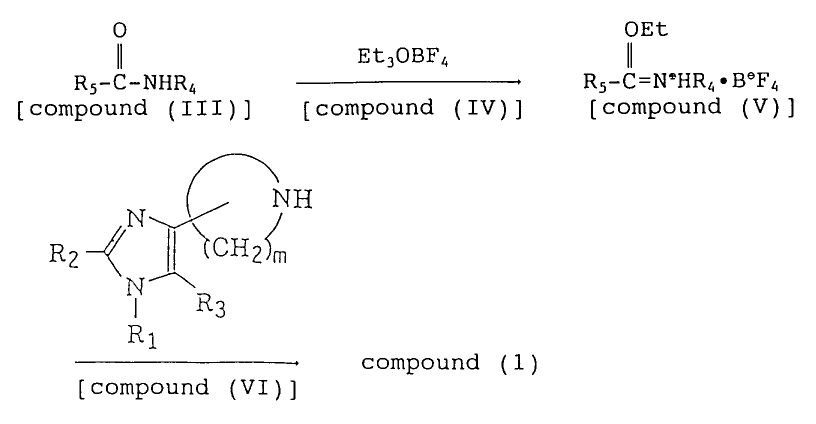 It is preferable to perform the reaction of the first step in an organic solvent such as dichloromethane. The reaction conditions are exemplified by a reaction temperature of room temperature and a reaction time ranging from 1 to 30 hours.
   It is preferable to perform the reaction of the second step in a solvent such as anhydrous ethanol. The reaction conditions are exemplified by a reaction temperature of room temperature and a reaction time ranging from 1 to 5 days.
   The term "the first step" and "the second step" as used herein respectively mean the former reaction step and the latter one. The same will apply hereinafter.
(3) 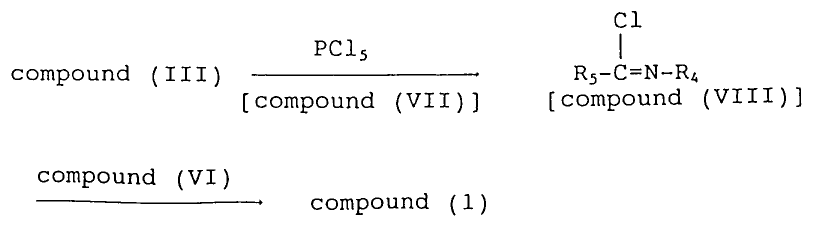 It is preferable to perform the reaction of the first step in an organic solvent such as benzene or xylene. The reaction conditions are exemplified by a reaction temperature ranging from 40 to 60 °C and a reaction time ranging from 10 minutes to 3 hours.
   It is also preferable to perform the reaction of the second step in an organic solvent such as benzene or xylene. The reaction conditions are exemplified by a reaction temperature ranging from 80 to 100 °C and a reaction time ranging from 1 to 10 hours.
(4) 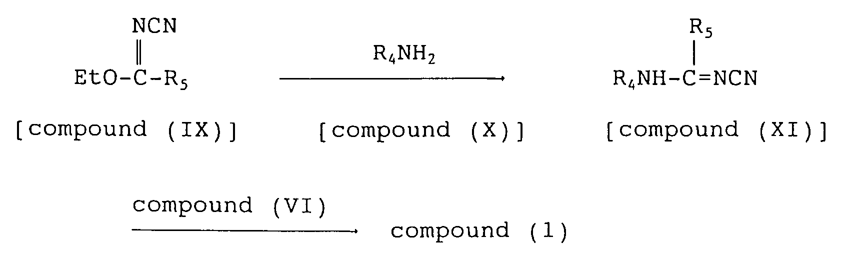 It is preferable to perform the reaction of the first step in a solvent such as ethanol. The reaction conditions are exemplified by a reaction temperature of room temperature and a reaction time ranging from 6 to 24 hours.
   It is preferable to perform the reaction of the second step in a polar solvent such as water, methanol or ethanol. The reaction conditions are exemplified by a reaction temperature of room temperature and a reaction time ranging from 1 minute to 24 hours.
(5) 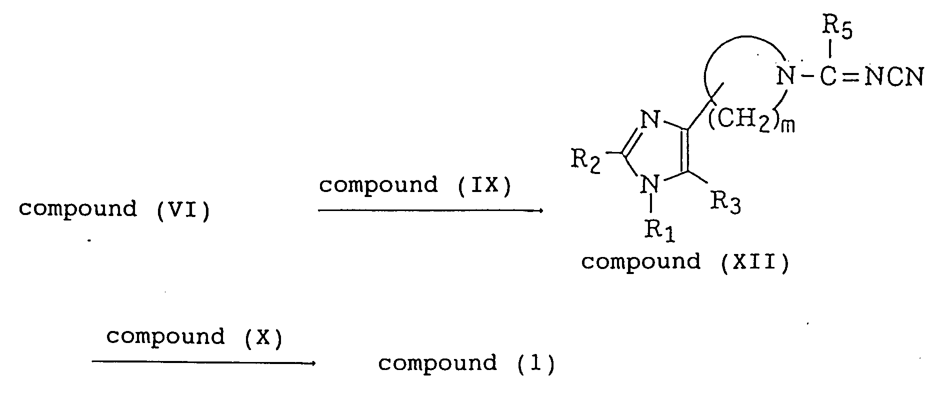 It is preferable to perform the reaction of the first step under the same reaction conditions as those of the first step of the method (4). Also, it is preferable to perform the reaction of the second step under the same reaction conditions as those of the second step of the method (4).
(6) 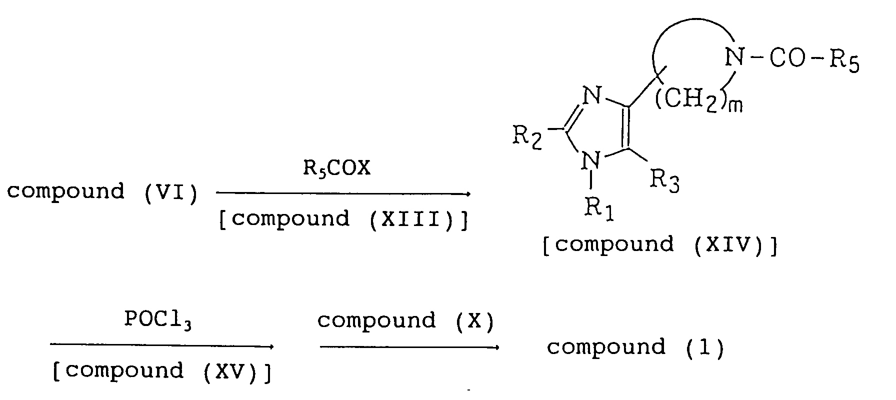 wherein X represents a halogen atom or OCOEt.
   The reaction of the first step is a usual amidation reaction, while the reaction of the second step is preferably performed in an organic solvent such as benzene. The reaction conditions are exemplified by a reaction temperature of room temperature and a reaction time ranging from 1 to 30 hours.
   It is also preferable to perform the reaction of the third step in an organic solvent such as benzene. The reaction conditions are exemplified by a reaction temperature ranging from 65 to 70 °C and a reaction time ranging from 1 to 15 hours.

The invention compound (2) may be synthesized in accordance with the following reaction scheme. 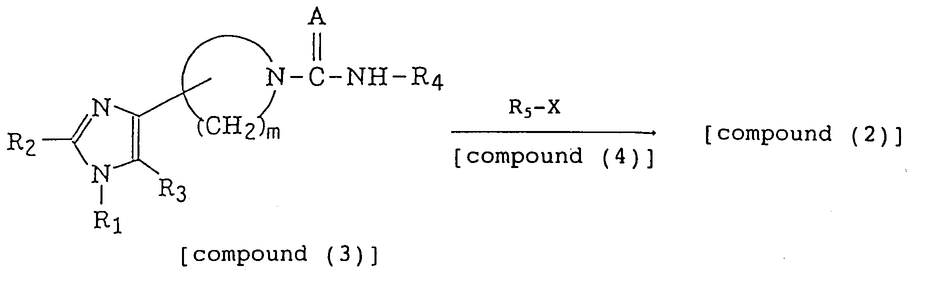
wherein X represents a halogen atom or a p-toluenesulfonyloxy group, or R₅-X represents (R₅)₂SO₄ or (R₅)₃O⁺BF₄⁻; and other substituents are each as defined above.

The compound (3) is a publicly known compound described in, for example, JP-A-61-267574 and EP-A-494010.

The invention compound (2) can be obtained by reacting the compound (3) with the compound (4). When X represents a halogen atom in the above reaction scheme, a preferable example thereof is an iodine atom.

When X represents a halogen atom or a p-toluene-sulfonyloxy group, it is preferable to perform the reaction in a methanol solution optionally containing 10 % of hydrogen chloride.

When R₅-X represents (R₅)₂SO₄, the reaction is performed, for example, without using any solvent or in benzene.

When R₅-X represents (R₅)₃O⁺BF₄⁻, the reaction is performed, for example, in a dichloromethane solution.

When X represents a halogen atom or a p-toluenesulfonyloxy group, the reaction conditions are exemplified by a reaction temperature ranging from ice-cooling to room temperature and a reaction time ranging from 1 to 10 hours.

When R₅-X represents (R₅)₂SO₄, the reaction conditions are exemplified by a reaction temperature ranging from 70 to 120 °C and a heating time ranging from 2 to 6 hours.

When R₅-X represents (R₅)₃O⁺BF₄⁻, the reaction conditions are exemplified by a reaction temperature ranging from 0 °C to room temperature and a reaction time ranging from 12 to 24 hours.

After the completion of the reaction, the obtained compounds (1) and (2) can be isolated and purified in the form of an acid addition salt or a free base by publicly known procedures, for example, recrystallization, TLC or adsorption chromatography. When such a compound is to be purified in the form of an acid addition salt, it is advantageous that its picrate is once formed and then converted into the desired acid addition salt.

The results of these studies indicate that the derivatives of the present invention have a low toxicity and advantageous antagonistic properties to a histamine H₃ receptor and exhibit an ability to increase the amount of released cerebral histamine. Because of having these characteristics, the invention compounds (1) and (2) are the first ones of this type which are highly useful in human medicine and veterinary medicine. The therapeutic application of these compounds relates to the central nervous system and peripheral organs which are under the control of the histamine H₃ receptor.

The drug of the present invention can be administered orally, intravenously, sublingually, nasally, rectally or extra-intestinally. The active ingredient may be formulated together with therapeutically appropriate fillers or liquid diluents. It is advantageous that each unit dose contains from 0.5 to 100 mg of the active ingredient, while the daily dose of the active ingredient may be varied within a range of from 0.5 to 200 mg.

### Best Mode for Practice of the Invention

To further illustrate the present invention in greater detail, the following examples will be given. However, it is to be understood that the present invention is not restricted thereto.

### Example 1

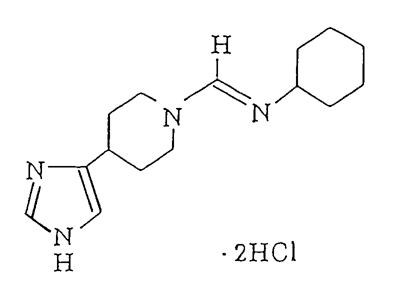

### Synthesis of N-cyclohexyl-N',N'-[1,5-[3-(4(5)-1H-imidazolyl)pentanediyl]]formamidin dihydrochloride

Raney nickel (about 1 g) was added to a solution of N-cyclohexyl-N',N'-[1,5-[3-(4(5)-1H-imidazolyl)pentanediyl]]thiourea (thioperamide) (200 mg) in ethanol (5 ml). After stirring the resulting mixture under ice-cooling, the supernatant was recovered by decantation. Then, the solvent was distilled off under reduced pressure and thus a white powder was obtained. This powder was dissolved in ethanol (5 ml) and a solution of hydrochloric acid in ethanol (5.6 N, 0.25 ml) was added thereto. After stirring the mixture under ice-cooling for 30 minutes, the solvent was distilled off under reduced pressure to thereby give the title compound (175 mg) in the form of a blue powder.

The reaction scheme is as follows: 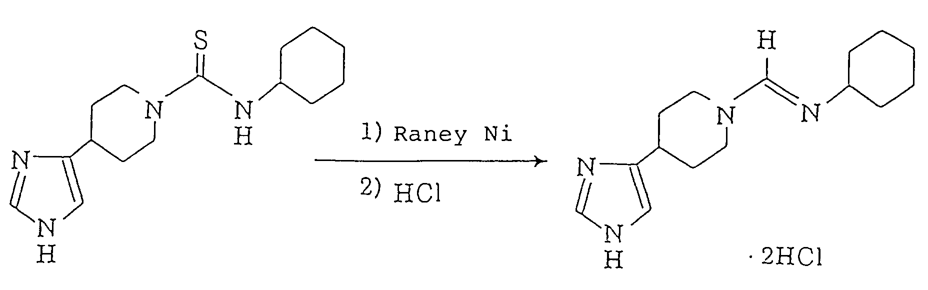
The analytical data of the title compound obtained above are as follows:
IR (KBr):
3400, 2920, 1690, 1620, 1450.
¹H-NMR (MeOH-d₄) δ:
8.89 (s, 1H),
8.09 (brs, 1H),
7.43 (s, 1H),
4.20 (brd, J = 13.5 Hz, 1H),
3.95 (brd, J = 12.9 Hz, 1H),
3.61 (td, J = 12.6, 2.7 Hz, 1H),
3.50 - 3.30 (m, 2H),
3.22 (tt, J = 11.8, 3.7 Hz, 1H),
2.40 - 2.10 (m, 2H),
2.10 - 1.60 (m, 7H),
1.60 - 1.10 (m, 5H).

### Example 2

### Synthesis of N-(1-adamantyl)-N' N'-[1,5-(3-(4(5)-1H-imidazolyl)pentanediyl]]formamidin dihydrochloride

Raney nickel (200 g) was added to a solution of N-(1-adamantyl)-N',N'-[1,5-[3-(4(5)-1H-imidazolyl)pentanediyl]]thiourea (13.2 g) in ethanol (200 ml) under ice-cooling, and the mixture was stirred for 1.5 hours. After the completion of the reaction, the supernatant was recovered by decantation and the residual Raney Ni was washed with ethanol. Then the supernatant was combined with the washing liquor and filtered. To the filtrate was added a solution of methanol containing 10 % of hydrogen chloride (300 ml). After stirring the mixture for 30 minutes, the solvent was distilled off to thereby give 10.5 g of a crude product of the above-mentioned compound.

A solution of the above-mentioned crude product (10.5 g) in methanol (140 ml) was added to a solution of picric acid (20.0 g) in methanol (350 ml). Then water (700 ml) was further added thereto. The yellow powder thus precipitated was recovered by filtration and recrystallized from methanol/acetone/ether. Thus, the picrate of the above-mentioned compound (8.7 g) was obtained in the form of a yellow powder.

The resulting picrate was mixed with 3 N hydrochloric acid, and the picric acid thus liberated was removed by using nitromethane followed by washing. Then, the aqueous layer was distilled off under reduced pressure, and the residue was recrystallized from ethanol/ether. Thus, 3.51 g of the title compound was obtained in the form of a white powder.
m.p.: 240 °C (decomp.)
IR (KBr):
2800, 1682, 1602, 1460, 1348, 1065, 950, 795, 600.
¹H-NMR (D₂O) δ:
8.62 (s, 1H),
7.80 (s, 1H),
7.29 (s, 1H),
4.07 (d, J = 13.9 Hz, 1H),
3.91 (d, J = 13.8 Hz, 1H),
3.58 (td, J = 13.3, 2.9 Hz, 1H),
3.28 (td, J = 13.3, 2.9 Hz, 1H),
3.21 (tt, J = 11.5, 3.6 Hz, 1H),
2.31 - 2.05 (m, 5H),
1.55 (m, 14H).

### Example 3

### Synthesis of N-cyclohexyl-N',N'-[1,5-[3-(4(5)-1H-imidazolyl)pentanediyl]]formamidin dihydrochloride

Starting from N-cyclohexyl-N',N'-[1,5-[3-(4(5)-1H-imidazolyl)pentanediyl]]thiourea (thioperamide), the procedure of Example 2 was repeated to thereby synthesize the title compound.
¹H-NMR (MeOH-d₄) δ:
8.89 (s, 1H),
8.09 (brs, 1H),
7.43 (s, 1H),
4.20 (brd, 13.5 Hz, 1H),
3.95 (brd, J = 12.9 Hz, 1H),
3.61 (td, J = 12.6, 2.7 Hz, 1H),
3.50 - 3.30 (m, 2H),
3.22 (tt, J = 11.8, 3.7 Hz, 1H),
2.40 - 2.10 (m, 2H),
2.10 - 1.60 (m, 7H),
1.60 - 1.10 (m, 5H).

### Example 4

### Synthesis of N-(exo-2-norbornyl)-N',N'-[1,5-[3-(4(5)-1H-imidazolyl)pentanediyl]]formamidin dihydrochloride

Starting from N-(exo-norbornyl)-N',N'-[1,5-[3-(4(5)-1H-imidazolyl)pentanediyl]]thiourea, the procedure of Example 2 was repeated to thereby synthesize the title compound.
¹H-NMR (D₂O) δ:
8.63 (d, J = 1.0 Hz, 1H),
7.88 (s, 1H),
7.30 (s, 1H),
4.05 (d, J = 14.2 Hz, 1H),
3.86 (d, J = 13.6 Hz, 1H),
3.75 - 3.47 (m, 2H),
3.40 - 3.12 (m, 2H),
2.42 - 2.28 (m, 2H),
2.21 (d, J = 11.7 Hz, 2H),
1.93 - 1.71 (m, 3H),
1.64 - 1.38 (m, 4H),
1.37 - 1.03 (m, 3H).

### Example 5

### Synthesis of N-(2,2-dimethyl-1-methylpropyl)-N',N'-[1,5-[3-(4(5)-1H-imidazolyl)pentanediyl]]formamidin dihydrochloride

Starting from N-(2,2-dimethyl-1-methylpropyl)-N',N'-[1,5-[3-(4(5)-1H-imidazolyl)pentanediyl]]thiourea, the procedure of Example 2 was repeated to thereby synthesize the title compound.
¹H-NMR (D₂O) δ:
8.64 (d, J = 1.4 Hz, 1H),
7.90 (d, J = 2.6 Hz, 1H),
7.30 (d, J = 1.0 Hz, 1H),
4.08 (d, J = 13.5 Hz, 1H),
3.89 (d, J = 13.3 Hz, 1H),
3.71 - 3.52 (m, 1H),
3.47 - 3.16 (m, 3H),
2.24 (d, J = 12.1 Hz, 2H),
1.96 - 1.67 (m, 2H),
1.28 (d, J = 6.9 Hz, 3H),
0.94 (s, 9H).

### Example 6

### Synthesis of N-(4-chlorobenzyl)-N',N'-[1,5-[3-(4(5)-1H-imidazolyl)pentanediyl]]formamidin dihydrochloride

Starting from N-(4-chlorobenzyl)-N',N'-[1,5-[3-(4(5)-1H-imidazolyl)pentanediyl]]thiourea, the procedure of Example 2 was repeated to thereby synthesize the title compound.
¹H-NMR (D₂O) δ:
8.65 (d, J = 1.3 Hz, 1H),
8.05 (s, 1H),
7.55 - 7.44 (m, 2H),
7.36 (d, J = 8.5 Hz, 2H),
7.31 (s, 1H),
4.63 (s, 2H),
4.12 - 3.84 (m, 2H),
3.77 - 3.57 (m, 1H),
3.38 (ddd, J = 12.8, 12.8, 2.9 Hz, 1H)
3.26 (dddd, J = 11.6, 11.6, 3.5, 3.5 Hz, 1H),
2.25 (d, J = 12.9 Hz, 2H),
1.99 - 1.68 (m, 2H).

### Example 7

### Synthesis of N-phenethyl-N',N'-[1,5-[3-(4(5)-1H-imidazolyl)pentanediyl]]formamidin dihydrochloride

Starting from N-phenethyl-N',N'-[1,5-[3-(4(5)-1H-imidazolyl)pentanediyl]]thiourea, the procedure of Example 2 was repeated to thereby synthesize the title compound.
¹H-NMR (D₂O) δ:
8.65 (d, J = 1.4 Hz, 1H),
7.55 - 7.22 (m, 7H),
3.98 - 3.82 (m, 1H),
3.82 - 3.54 (m, 3H),
3.54 - 3.36 (m, 1H),
3.36 - 3.04 (m, 2H),
3.04 - 2.80 (m, 2H),
2.32 - 2.04 (m, 2H),
1.77 - 1.40 (m, 2H).

### Example 8

### Synthesis of N-(3-fluorophenyl)-N',N'-[1,5-[3-(4(5)-1H-imidazolyl)pentanediyl]]formamidin dimaleate

Raney nickel (150 g) was added to a solution of N-(3-fluorophenyl)-N',N'-[1,5-[3-(4(5)-1H-imidazolyl)pentanediyl]]thiourea (10.1 g) in ethanol (400 ml) under ice-cooling, and the mixture was stirred for 4 hours. After the completion of the reaction, the supernatant was recovered by decantation, and the residual Raney Ni was washed with ethanol. Then, the supernatant was combined with the washing liquor and filtered. The solvent was distilled off from the filtrate to thereby give 4.93 g of the free base of the above-mentioned compound in the form of a pale yellow powder.

A solution of maleic acid (4.17 g) in ethanol (20 ml) was added dropwise into a solution of the free base (4.73 g) in ethanol (20 ml). After stirring the mixture at room temperature for 30 minutes, ether (50 ml) was added thereto. The white powder thus precipitated was recovered by filtration. Thus, 7.54 g of the title compound was obtained in the form of a white powder.
m.p.: 148 - 150 °C.
IR (KBr):
3400, 2950, 2800, 1690, 1570, 1470, 1360, 1190, 985.
¹H-NMR (D₂O) δ:
8.60 (d, J = 1.3 Hz, 1H),
8.40 (s, 1H),
7.50 - 7.35 (m, 1H),
7.14 - 6.98 (m, 3H),
6.22 (s, 4H),
4.26 (brd, J = 14.0 Hz, 1H),
4.05 (brd, J = 13.6 Hz, 1H),
3.74 (ddd, J = 12.9, 12.9, 2.8 Hz, 1H),
3.48 (ddd, J = 12.9, 12.9, 2.4 Hz, 1H),
3.25 (tt, J = 11.6, 3.6 Hz, 1H),
2.27 (brd, J = 12.7 Hz, 2H),
1.94 (ddd, J = 12.3, 8.3, 4.2 Hz, 1H),
1.81 (ddd, J = 12.5, 8.5, 4.2 Hz, 1H).

### Example 9

### Synthesis of N-phenyl-N',N'-[1,5-[3-(4(5)-1H-imidazolyl)pentanediyl]]formamidin

Raney nickel (about 4 g) was added to a solution of N-phenyl-N',N'-[1,5-[3-(4(5)-1H-imidazolyl)pentanediyl]]thiourea (572 mg) in ethanol (25 ml) under ice-cooling, and the mixture was stirred for 2.5 hours. After the completion of the reaction, the supernatant was recovered by decantation, and the residual Raney Ni was washed with ethanol. Then, the supernatant was combined with the washing liquor and filtered. After distilling off the solvent, a crude product of the above-mentioned compound was obtained. This crude product was subjected to silica gel column chromatography (chloroform/methanol = 10/1 → 5/1 → 3/1). Thus, 211 mg of the title compound was obtained in the form of a foamy substance.
IR (KBr):
3620 - 2380, 1616, 1582, 760, 695.
¹H-NMR (DMSO-d₆) δ:
12.96 - 10.67 (brs, 1H),
7.73 (s, 1H),
7.52 (s, 1H),
7.20 (t, J = 7.8 Hz, 2H),
6.98 - 6.86 (m, 3H),
6.78 (s, 1H),
4.67 - 4.03 (m, 1H),
4.03 - 3.62 (m, 1H),
3.62 - 2.87 (m, 2H),
2.87 - 2.66 (m, 1H),
2.04 - 1.83 (m, 1H),
1.50 (dddd, J = 12.2, 12.2, 12.2, 4.1 Hz, 2H).

### Test Example 1

The affinity of the compound of the present invention obtained in the above Examples for the histamine H₃ receptor was examined in a binding test using rat cerebral cortex membrane and [³H] (R)-α-methylhistamine. The invention compounds (1) showed Ki values (dissociation constant to the histamine H₃ receptor) of from 5 to 200 nM. When used in an amount of 1 mM, every invention compound scarcely showed any histamine-induced contraction in isolated guinea pig ileum mediated by the histamine H₁ receptor [Ash et al., Br. J. Pharmac. Chemothera., 27, 427 - 439 (1966)] or any histamine-induced positive chronotropism of isolated guinea pig right atrium mediated by the histamine H₂ receptor [Black et al., Nature, 236, 385 - 390 (1972)]. These results indicate that the invention compound (1) has a high selectivity for the histamine H₃ receptor.

### Example 10

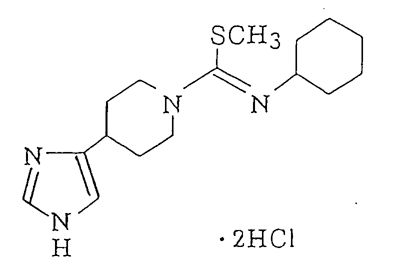

### Synthesis of N-cyclohexyl-N',N'-[1,5-[3-(4(5)-1H-imidazolyl)pentanediyl]]-S-methylisothiourea dihydrochloride

Iodomethane (4.85 g) was added to a solution of N-cyclohexyl-N',N'-[1,5-[3-(4(5)-1H-imidazolyl)pentanediyl]]thiourea (thioperamide) (500 mg) in a 10 % hydrochloric acid-containing methanol (20 ml), and the mixture was stirred under ice-cooling for 5 hours. After distilling off the reaction solvent under reduced pressure, the residue was dissolved in ethanol and treated with activated charcoal. Then, it was recrystallized from ethanol/ether. Thus, the title compound (339 mg) was obtained in the form of a yellow powder.
m.p.: 145 °C (decomp.)
IR (KBr):
3400, 2900, 1590, 1450, 1400.
¹H-NMR (MeOH-d₄) δ:
8.85 (s, 1H),
7.43 (s, 1H),
4.25 (d, J = 13.7 Hz, 2H),
3.98 (tt, J = 11, 4.0 Hz, 1H),
3.57 (td, J = 13.4, 2.5 Hz, 2H),
3.25 (tt, J = 11, 4 Hz, 1H),
2.65 (s, 3H),
2.32 - 2.10 (m, 2H),
2.05 - 1.10 (m, 12H).

### Example 11

### Synthesis of N-cyclohexyl-N',N'-[1,5-[3-(4(5)-1H-imidazolyl)pentanediyl]]-S-methylisothiourea dimaleate

The title compound was synthesized in accordance with the method of Example 10.
White powder (m.p.: 138 - 141 °C).
IR (KBr):
1580, 1490, 1390, 1370, 1200, 1080, 990, 865.
¹H-NMR (MeOH-d₄) δ:
8.64 (s, 1H),
7.31 (s, 1H),
6.26 (s, 4H),
4.24 (d, J = 13.8 Hz, 2H),
4.05 - 3.90 (m, 1H),
3.53 (td, J = 11.3, 2.0 Hz, 2H),
3.19 (tt, J = 11.7, 3.95 Hz, 1H),
2.64 (s, 3H),
2.25 - 2.15 (m, 2H),
2.08 - 1.65 (m, 7H),
1.65 - 1.10 (m, 5H).

### Example 12

### Synthesis of N-(exo-2-norbornyl)-N',N'-[1,5-[3-(4(5)-1H-imidazolyl)pentanediyl]]-S-methylisothiourea dihydrochloride

Iodomethane (41 ml) was added to a solution comprising N-(exo-2-norbornyl)-N',N'-[1,5-[3-(4(5)-1H-imidazolyl)pentanediyl]]thiourea (11.6 g) and 10 % hydrogen chloride-containing methanol solution (580 ml) under ice-cooling. Then, the reaction mixture was stirred successively under ice-cooling for 5 hours and at room temperature for 2 hours. After distilling off the reaction solvent under reduced pressure, the residue was dissolved in methanol (150 ml). Then, it was added to a solution of picric acid (28.8 g) dissolved in methanol (400 ml), and then water (1200 ml) was further added thereto. The yellow powder thus precipitated was recovered by filtration and then recrystallized from acetone/ether to thereby give 21.9 g of the picrate of the above-mentioned compound in the form of a yellow powder. This powder was added to 3 N hydrochloric acid (650 ml), and the picric acid thus liberated was removed by using nitromethane followed by washing. The aqueous layer was distilled off under reduced pressure, and the residue thus obtained was recrystallized from ethanol/ether. Thus, 8.77 g of the title compound was obtained in the form of white crystals.
m.p.: 170.5 - 171.5 °C.
IR (KBr):
3650 - 3225, 3225 - 2660, 1595, 1448, 1387, 1360, 1255,
1093, 824.
¹H-NMR (D₂O) δ:
8.66 (d, J = 1.3 Hz, 1H),
7.33 (s, 1H),
4.24 (d, J = 13.6 Hz, 2H),
3.98 - 3.88 (m, 1H),
3.63 - 3.48 (m, 2H),
3.27 (dddd, J = 3.7, 3.7, 11.7, 11.7 Hz, 1H),
2.62 (s, 3H),
2.42 - 2.33 (m, 2H),
2.32 - 2.22 (m, 2H),
1.97 - 1.80 (m, 3H),
1.66 - 1.46 (m, 4H),
1.35 - 1.24 (m, 2H),
1.23 - 1.14 (m, 1H).

### Example 13

### Synthesis of N-(1-admantyl)-N',N'-[1,5-[3-(4(5)-1H-imidazolyl)pentanediyl]]-S-methylisothiourea dihydrochloride

Starting from N-(1-admantyl)-N',N'-[1,5-[3-(4(5)-1H-imidazolyl)pentanediyl]]thiourea, the title compound was synthesized in accordance with the method of Example 12.
¹H-NMR (DMSO-d₆) δ:
15.16 - 15.01 (brs, 1H),
14.55 - 14.33 (brs, 1H),
9.09 (s, 1H),
8.98 (s, 1H),
7.49 (s, 1H),
4.09 (d, J = 14.2 Hz, 2H),
3.62 - 3.35 (m, 2H),
3.27 - 3.07 (m, 1H),
2.64 (s, 3H),
2.28 - 2.00 (m, 1H),
2.00 - 1.74 (m, 2H),
1.74 - 1.60 (m, 6H).

### Example 14

### Synthesis of N-(2,2-dimethyl-1-methylpropyl)-N',N'-[1,5-[3-(4(5)-1H-imidazolyl)pentanediyl]]-S-methylisothiourea dihydrochloride

Starting from N-(2,2-dimethyl-1-methylpropyl)-N',N'-[1,5-[3-(4(5)-1H-imidazolyl)pentanediyl]]thiourea, the title compound was synthesized in accordance with the method of Example 12.
¹H-NMR (D₂O) δ:
8.65 (d, J = 1.4 Hz, 1H),
7.30 (3, 1H),
4.46 - 4.13 (m, 3H),
3.68 - 3.46 (m, 2H),
3.38 - 3.19 (m, 1H),
2.60 (s, 3H),
2.27 (d, J = 13.0 Hz, 2H),
1.98 - 1.71 (m, 2H),
1.30 (d, J = 6.9 Hz, 3H),
0.97 (s, 9H).

### Example 15

### Synthesis of N-(4-chlorobenzyl)-N',N'-[1,5-[3-(4(5)-1H-imidazolyl)pentanediyl]]-S-methylisothiourea dihydrochloride

Starting from N-(4-chlorobenzyl)-N',N'-[1,5-[3-(4(5)-1H-imidazolyl)pentanediyl]]thiourea, the title compound was synthesized in accordance with the method of Example 12.
¹H-NMR (D₂O) δ:
8.65 (d, J = 1.3 Hz, 1H),
7.48 (d, J = 8.5 Hz, 2H),
7.36 (d, J = 8.5 Hz, 2H),
7.31 (s, 1H),
4.86 (s, 2H),
4.30 (d, J = 14.0 Hz, 2H),
3.73 - 3.49 (m, 2H),
3.41 - 3.18 (m, 1H),
2.55 (s, 3H),
2.37 - 2.19 (m, 2H),
2.00 - 1.72 (m, 2H).

### Example 16

### Synthesis of N-phenethyl-N',N'-[1,5-[3-(4(5)-1H-imidazolyl)pentanediyl]]-S-methylisothiourea dihydrochloride

Starting from N-phenethyl-N',N'-[1,5-[3-(4(5)-1H-imidazolyl)pentanediyl]]thiourea, the title compound was synthesized in accordance with the method of Example 12.
¹H-NMR (D₂O) δ:
8.64 (d, J = 1.3 Hz, 1H),
7.51 - 7.24 (m, 6H),
4.18 - 3.96 (m, 4H),
3.42 (t, J = 12.3 Hz, 2H),
3.30 - 3.10 (m, 1H),
3.01 (t, J = 6.5 Hz, 2H),
2.31 (s, 3H),
2.27 - 2.11 (s, 2H),
1.85 - 1.54 (m, 2H).

### Example 17

### Synthesis of N-(3-fluorophenyl)-N',N'-[1,5-[3-(4(5)-1H-imidazolyl)pentanediyl]]-S-methylisothiourea

Iodomethane (1.0 ml) was added to a solution comprising N-(3-fluorophenyl)-N',N'-[1,5-[3-(4(5)-1H-imidazolyl)pentanediyl]]thiourea (456 mg) and a 10 % hydrogen chloride-containing methanol solution (20 ml) under ice-cooling. Then, the reaction mixture was stirred under ice-cooling for 5 hours. After distilling off the reaction solvent under reduced pressure, the resulting residue was dissolved in methanol (150 ml). Then, a saturated aqueous solution of sodium hydrogencarbonate was added thereto, and the mixture was extracted with chloroform. The chloroform layer was washed with a saturated aqueous solution of sodium chloride and dried over anhydrous magnesium sulfate. After distilling off the solvent, the residue was subjected to silica gel column chromatography (chloroform/methanol = 20/1 → 10/1). Thus, 381 mg of the title compound was obtained in the form of a resinous material.
IR (KBr):
3060, 2920, 2830, 1563, 1182, 1116, 1093, 772, 748, 692.
¹H-NMR (DMSO-d₆) δ:
11.93 (brs, 0.4H),
11.80 (brs, 0.6H),
7.53 (s, 1H),
7.24 (dd, J = 8.2 Hz, 15.2 Hz, 1H),
6.85 (brs, 1H),
6.73 (dt, J = 2.0 Hz, 8.3 Hz, 1H),
6.66 - 6.49 (m, 2H),
3.04 (t, J = 11.5 Hz, 2H),
2.11 (s, 3H),
1.95 (d, J = 10.8 Hz, 2H),
1.58 (q, J = 11.7 Hz, 2H).

### Example 18

### Synthesis of N-phenyl-N',N'-[1,5-[3-(4(5)-1H-imidazolyl)pentanediyl]]-S-methylisothiourea

Starting from N-phenyl-N',N'-[1,5-[3-(4(5)-1H-imidazolyl)pentanediyl]]thiourea, the title compound was synthesized in accordance with the method of Example 17.
¹H-NMR (DMSO-d₆) δ:
12.24 - 11.43 (brs, 1H),
7.52 (s, 1H),
7.22 (t, J = 7.7 Hz, 2H),
6.93 (t, J = 7.3 Hz, 1H),
6.77 (s, 1H),
6.76 (d, J = 7.3 Hz, 2H),
4.15 (d, J = 13.0 Hz, 2H),
3.00 (t, J = 12.0 Hz, 2H),
2.88 - 2.66 (m, 1H),
2.09 (s, 3H),
1.96 (d, J = 12.6 Hz, 2H),
1.58 (dq, J = 3.2 Hz, 12.3 Hz, 2H).

### Test Example 2

The affinity of the compound of the present invention obtained in the above Examples for the histamine H₃ receptor was examined in a binding test using rat cerebral cortex membrane and [³H] (R)-α-methylhistamine. The invention compounds (2) showed Ki values (dissociation constant to the histamine H₃ receptor) of from 40 to 700 nM. When used in an amount of 1 mM, every invention compound scarcely showed any histamine-induced contraction in isolated guinea pig ileum mediated by the histamine H₁ receptor [Ash et al., Br. J. Pharmac. Chemothera., 27, 427 - 439 (1966)] or any histamine-induced positive chronotropism of isolated guinea pig right atrium mediated by the histamine H₂ receptor [Black et al., Nature, 236, 385 - 390 (1972)]. These results indicate that the invention compound (2) has a high selectivity for the histamine H₃ receptor.

### Industrial Applicability

The imidazole-series compounds of the present invention are usable as a psycho-activator, a sleep regulator, a cerebral metabolism activator aiming at treating Alzheimer's disease, etc., an anticonvulsant, an analgesic, a feeding regulator, a thermoregulator, an endocrine regulator, etc. They are also usable as a labeling compound for the image processing of the histamine H₃ receptor by PET (positron emission tomography).

## Claims

1. A compound represented by the following general formula (0): 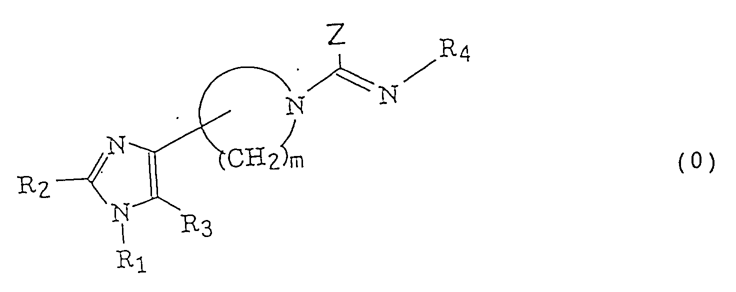 wherein m represents an integer of from 4 to 6; R₁ represents a hydrogen atom or a lower alkyl or aralkyl group; R₂ and R₃ may be either the same or different from each other and each represents a hydrogen atom or a lower alkyl group; R₄ represents a hydrogen atom, a linear or branched alkyl group, a cycloalkyl group, a cycloalkylalkyl group, a substituted or unsubstituted aryl group or a substituted or unsubstituted aralkyl group; and Z represents R₅ or A-R₆, wherein A represents S or O, R₅ represents a hydrogen atom, a lower alkyl group, a substituted or unsubstituted aryl group or a substituted or unsubstituted aralkyl group, and R₆ represents a lower alkyl group, a lower alkenyl group, a lower alkynyl group or a substituted or unsubstituted aralkyl group; or a pharmaceutically acceptable salt thereof.

2. A compound represented by the following general formula (1): 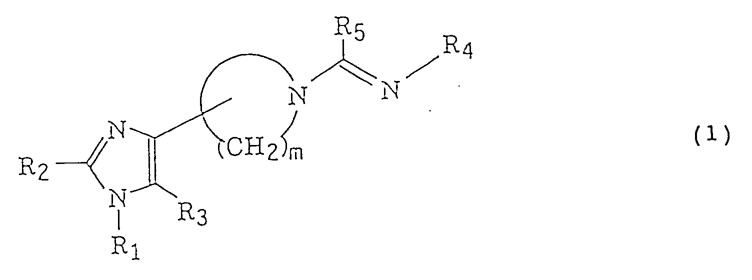 wherein m and R₁ to R₄ are each as defined above; or a pharmaceutically acceptable salt thereof.

3. A compound represented by the following general formula (2): 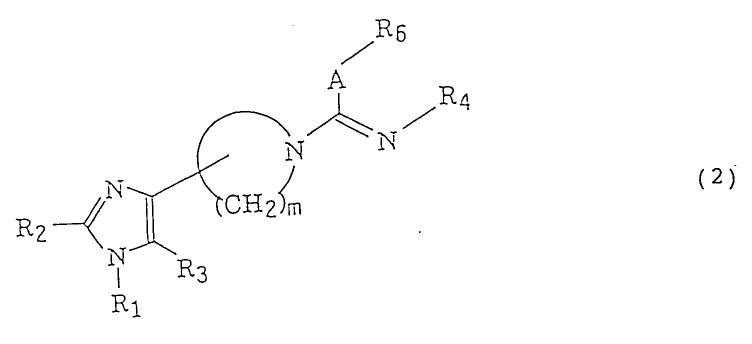 wherein A, m, R₁ to R₄ and R₆ are each as defined above; or a pharmaceutically acceptable salt thereof.

4. A compound as claimed in claim 1, wherein m is 5 and R₁, R₂ and R₃ are each a hydrogen atom.

5. A compound as claimed in claim 1, wherein R₄ is a cycloalkyl group.

6. A compound as claimed in claim 5, wherein the cycloalkyl group is a monocycloalkyl, bicycloalkyl or tricycloalkyl group.

7. A compound as claimed in claim 6, wherein the monocycloalkyl group is a cyclohexyl group.

8. A compound as claimed in claim 7, wherein the bicycloalkyl group is a norbornyl group.

9. A compound as claimed in claim 8, wherein the norbornyl group is a 2-exo-norbornyl group.

10. A compound as claimed in claim 6, wherein the tricycloalkyl group is an adamantyl group.

11. A compound as claimed in claim 10, wherein the adamantyl group is a 1-adamantyl group.

12. A compound as claimed in claim 1, wherein R₄ is a substituted or unsubstituted phenyl group.

13. A compound as claimed in claim 1, wherein R₄ is a substituted or unsubstituted phenylalkyl group.

14. A compound as claimed in claim 1, wherein R₅ is a hydrogen atom.

15. A compound as claimed in claim 1, wherein A is S and R₆ is a lower alkyl group.

16. A compound as claimed in claim 15, wherein the lower alkyl group is a methyl group.

17. A compound as claimed in claim 1, wherein R₁ represents a hydrogen atom, an alkyl group having 1 to 6 carbon atoms or an aralkyl group carrying an alkyl moiety having 1 to 4 carbon atoms; R₂ and R₃ each represents a hydrogen atom or an alkyl group having 1 to 6 carbon atoms; R₄ represents a hydrogen atom, a linear or branched alkyl group having 1 to 8 carbon atoms, a cycloalkyl group having 3 to 10 carbon atoms, a cycloalkylalkyl group composed of a cycloalkyl moiety having 3 to 10 carbon atoms and an alkyl moiety having 1 to 3 carbon atoms, a substituted or unsubstituted aryl group or a substituted or unsubstituted aralkyl group carrying an alkyl moiety having 1 to 4 carbon atoms; R₅ represents a hydrogen atom, an alkyl group having 1 to 6 carbon atoms, a substituted or unsubstituted aryl group or a substituted or unsubstituted aralkyl group carrying an alkyl moiety having 1 to 4 carbon atoms; and R₆ represents an alkyl group having 1 to 6 carbon atoms, an alkenyl group having 3 to 6 carbon atoms, an alkynyl group having 3 to 6 carbon atoms or a substituted or unsubstituted aryl group.

18. A compound as claimed in claim 1 which is one selected from among the following compounds:
N-cyclohexyl-N',N'-[1,5-[3-(4(5)-1H-imidazolyl)pentanediyl]]formamidin;
N-(1-adamantyl)-N',N'-[1,5-[3-(4-(5)-1H-imidazolyl)pentanediyl]]formamidin;
N-(exo-2-norbornyl)-N',N'-[1,5-[3-(4(5)-1H-imidazolyl)pentanediyl]]formamidin;
N-(2,2-dimethyl-1-methylpropyl)-N',N'-[1,5-[3-(4(5)-1H-imidazolyl)pentanediyl]]formamidin;
N-(4-chlorobenzyl)-N',N'-[1,5-[3-(4(5)-1H-imidazolyl)pentanediyl]]formamidin;
N-(phenethyl)-N',N'-[1,5-[3-(4(5)-1H-imidazolyl)pentanediyl]]formamidin;
N-(3-fluorophenyl)-N',N'-[1,5-[3-(4(5)-1H-imidazolyl)pentanediyl]]formamidin;
N-phenyl-N',N'-[1,5-[3-(4(5)-1H-imidazolyl)pentanediyl]]formamidin;
N-cyclohexyl-N',N'-[1,5-[3-(4(5)-1H-imidazolyl)pentanediyl]]-S-methylisothiourea;
N-(1-adamantyl)-N',N'-[1,5-[3-(4(5)-1H-imidazolyl)pentanediyl]]-S-methylisothiourea;
N-(exo-2-norbornyl)-N',N'-[1,5-[3-(4(5)-1H-imidazolyl)pentanediyl]]-S-methylisothiourea;
N-(2,2-dimethyl-1-methylpropyl)-N',N'-[1,5-[3-(4(5)-1H-imidazolyl)pentanediyl]]-S-methylisothiourea;
N-(4-chlorobenzyl)-N',N'-[1,5-[3-(4(5)-1H-imidazolyl)pentanediyl]]-S-methylisothiourea;
N-(phenethyl)-N',N'-[1,5-[3-(4(5)-1H-imidazolyl)pentanediyl]]-S-methylisothiourea;
N-(3-fluorophenyl)-N',N'-[1,5-[3-(4(5)-1H-imidazolyl)pentanediyl]]-S-methyl isothiourea; and
N-phenyl-N',N'-[1,5-[3-(4(5)-1H-imidazolyl)pentanediyl]]-S-methylisothiourea.
